# EUROPEAN PATENT APPLICATION

(11) **EP 0 796 588 A1**
(43) Date of publication of application: **24.09.1997**
(21) Application number: 97102351.0
(22) Date of filing: 13.02.1997
(51) Int. Cl.: A61B 5/00

(54) **Method and system for transferring physiological data to a data processing station**

(30) Priority: 20.03.1996 SE 9601064
(71) Applicant: Siemens-Elema AB, 171 95 Solna 1 (SE)
(72) Inventor: Emtell, Pär, 161 46 Bromma (SE); Kock, Mikael, 184 91 Akersberga (SE); Bergvist, Rune, 185 94 Vaxholm (SE)

(57) **Abstract**

In a method for transferring physiological data, obtained from a patient (3) with a stationary measuring device located by the patient and stored in a memory (8) in the measuring device, to a stationary data processing station (16), located at a distance from the patient, for processing and storage in a patient data base, data stored in the measuring device (2) are retrieved with a portable reading device (10) and temporarily stored in a memory (14) arranged therein. The portable reading device is then physically moved to or near the data processing station, and data stored in the reading device are downloaded to a memory (18) in the data processing station.

## Description

The present invention relates to a method for transferring physiological data, obtained from a patient with a stationary measuring device placed near the patient, said data being stored in a memory arranged in the measuring device, to a stationary data processing station, located at a distance from the measuring device, for processing and storage in a patient data base and a system for said data transfer.

Different ways of transferring physiological data, obtained from a patient, to a data processing station located at a distance from the patient are previously known. Thus, EP,A2,0 212 278 and WP 93/19667 describe systems for monitoring physiological data, obtained from a patient with a portable device, which, via a communications link such as a telephone line, is connected to a central station at a distance from the patient. EP,A2,0 601 589 and the brochure SIESTOLE, Holter ECG, "Recording the life in your patient's day", published by Siemens-Elema AB, describe systems in which a portable patient monitoring unit can be connected to a docking station or the like for transferring data to a remote station. European patent application 95 112 957.6 also describes the way in which data, recorded with a portable Holter ECG recorder and recorded on a cassette in the device, are transferred to separate playback and analysis equipment by removing the cassette from the Holter ECG unit and loading it in playback and analysis equipment so recorded data can be read.

DE,A1,43 15 532 describes a system using a portable device for measuring and storing a plurality of physiological measurement values, such as blood sugar levels, said device being connectable with special contact means to a data processing device for processing and visualizing the measurement values.

The transfer of measured data, e.g. via a cable from stationary measuring devices, connected to the patient, for recording physiological data from the patient, to a computer for storage and processing is also known. Alternately, measured data can be manually read and then entered, also manually, into a data processing device or patient records.

The object of the present invention is to set forth a new method for convenient transfer of physiological data, obtained from a patient, to a data processing station located at a distance from the patient and her/his stationary measuring device, as well as achieve a method for said data transfer.

This object is achieved with a method and system of the kind stated initially and with the features set forth in patent claims 1 and 2.

The present invention therefore permits convenient transfer of data, obtained from a patient with stationary equipment by the patient, to a stationary data processing unit located at distance therefrom. The invention can also be advantageously used for transferring data, measured in an ambulance during transport of the patient, to appropriate equipment when the ambulance arrives at hospital, enabling a physician to study the measured data and the results of any data processing.

According to an advantageous embodiment of the system according to the invention, the reading device contains an audio section with a microphone for entry of verbal information, such as comments by the physician, and an A/D converter for converting audio information into digital form for storage in the digital memory.

According to another advantageous embodiment of the system according to the invention, the reading device is arranged for downloading and temporary storage of data from a plurality of measuring devices, the temporarily stored data from the different measuring devices being readable by the data processing station in a single sequential read operation. In this manner, a plurality of measuring devices at the bedsides of a plurality of patients can be read with one and the same reading device when the physician makes her/his rounds. Here, the physician can advantageously record comments after reading each measuring device to keep track of the measuring devices which have been read.

According to yet another advantageous embodiment of the system according to the invention, the reading device is equipped with a flash memory which retains stored information even when the measuring device's source of energy is depleted or disabled.

According to another advantageous embodiment of the system according to the invention, the reading device contains a transmitter/receiver unit and the measuring device, the transmitter/receiver unit and data processing unit contain means for wireless input and output of data, preferably by means of telemetry or an IR link. In this manner, data transfer can be performed smoothly and conveniently.

According to a further advantageous embodiment of the system according to the invention, the reading device is devised for loading in a rack in the data processing station, during the downloading of data into the data processing station, for simultaneous recharging of the reading device's battery. A coil can e.g. be arranged to encircle the reading device, when it is loaded in the rack in the data processing station, to recharge the reading device's battery by electromagnetic means when voltage is applied to the coil, or the poles of the reading device's battery can be connected to external contacts which are devised, in turn, to connect to charging contacts in the data processing station when the reading device is loaded in the rack.

According to yet another advantageous embodiment of the system according to the invention, the reading device is incorporated into a penlight or devised as a pen-like implement. In this manner, the reading device can be conveniently carried in a breast pocket. A penlight and pen are otherwise instruments e.g. doctors and paramedics normally have with them. An additional advantage of combining the reading device with a penlight is that the penlight uses a battery which can also be used as a source of energy for the reading device.

According to yet another advantageous embodiment of the system according to the invention, the reading device is equipped with operating means for controlling input and output operations. Here, the button for triggering data retrieval or queries to the measuring device can be combined with e.g. the penlight's ON/OFF switch.

In order to explain the invention more thoroughly, an exemplifying embodiment of the system according to the invention will now be described in greater detail, referring to the attached drawing in which
FIG. 1 schematically illustrates one embodiment of the system according to the invention;
FIG. 2 shows an embodiment of the reading device in which the device has been combined with a penlight;
FIG. 3 shows a block diagram of the audio section of the reading device in the system according to the invention.

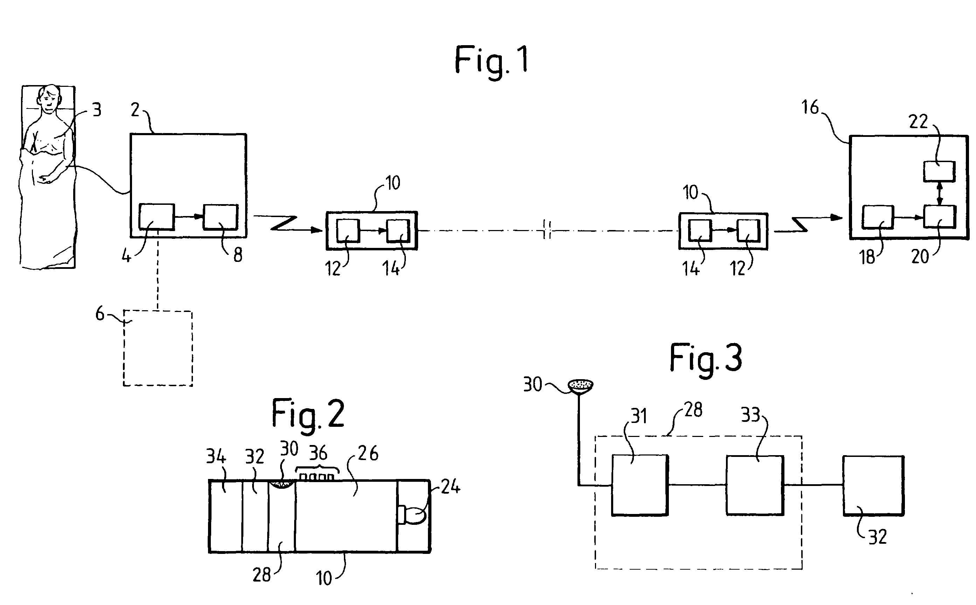

FIG. 1 shows a stationary measuring device 2, intended for placement e.g. at the patient's bedside, connected to the patient 3 in order to measure the her/his physiological data, such as parameters for the patient's respiration. Recorded data are stored in a memory 4 in the measuring device 2.

In hitherto known devices of the present kind, this memory is in a computer 6, or the computer 6 is connected to the memory 4 to process the physiological data and is illustrated with dashed lines in FIG. 1.

In a system according to the invention, the measuring device 2 also contains a unit 8, connected to the memory 4, for wireless transfer of stored data to a portable reading device 10. The reading device 10 contains a transmitter/receiver unit 12 connected to a memory 14.

The wireless transfer can be by telemetry or with the aid of an IR technic.

The reading device 10 is conveniently portable, and data stored in the reading device 10 are transferred to a station 16 for data processing and storage when the reading device 10 is physically brought into the vicinity of the processing station 16 for downloading of data stored in the device.

The data processing station 16 contains a receiver unit 18 for wireless downloading into memory 14 of stored data and data transferred by the reading device's 10 transmitter/receiver unit 12. The receiver unit 18 is further connected to a memory 20 and a processor 22.

In systems, according to the invention, designed for large hospitals, a plurality of such stations 16 are advantageously connected to a central computer in which patient data bases are stored.

The data processing station can hold patient data bases and/or patient medical records for storage of measurement values and/or measuring device settings.

As noted above, the reading device 10 is devised as a small, rugged, easily portable device. It can be combined to advantage with e.g. a penlight, an item physicians, paramedics etc. normally carry in their jobs. One advantage of such a combination is the fact that a penlight has batteries which can also be used for powering electronics equipment in the reading device 10. One example of this reading device design is shown in FIG. 2.

Thus, the reading device contains a bulb 24, a battery 26 and an audio unit 28 with a microphone 30, with which the physician is able to record comments and other verbal information.

The audio section 28 contains an A/D converter 31 connected to a processor 33 for converting recorded verbal information into digital form for storage in the following digital memory 32. Also see FIG. 3. The digital memory 32 is connected, in turn, to a transmitter/receiver unit 34, corresponding to the unit 12 in the system shown in FIG. 1.

The memory 32 therefore holds data transferred from the measuring device 2 and verbal information recorded by the reading device's user.

The reading device 10 further has operating buttons 36, e.g. an ON button to turn on the penlight, a recording button for controlling the transfer of information from the measuring device 2, a playback button for downloading data stored in the memory 32 to the data processing station 16 and an audio button for recording verbal information. The control button for triggering reading can be combined with e.g. the penlight's ON button. With these control buttons 36, the transmitter unit 8, for example, in the measuring device 2 can be triggered to transfer information, stored in the memory 4, to the reading device 10 and transfer this information, when stored in the memory 14 (or the memory 32 in the embodiment shown in FIG. 2) of the reading device 10, to the storage and processing station 16 on a subsequent occasion.

The memory 14, 32 of the reading device 10 is preferably a flash memory which preserves information, even if the battery become depleted or the reading device's source of energy fails for some reason.

The reading device 10 and the data processing station 16 are appropriately devised so the reading device's battery 26 is recharged at the same time as information is downloaded from the reading device's memory 14 to the data processing station 16. Thus, a coil can be arranged to encircle the penlight or battery 26, when the reading device 10 is loaded in a rack in the data processing station 16, to charge the reading device's 10 battery 26 by electromagnetic means by applying a voltage to the coil. Alternately, the poles of the reading device's 10 battery 26 can be connected to external contacts in the reading device's base or lateral walls which, in turn, make contact with recharge contacts in the data processing station 16 when the reading device 10 is loaded in the rack.

In the system according to the invention, the medical measuring device 2 is therefore scanned externally with e.g. a combined penlight, pocket memory and reader.

A plurality of measuring devices can be read with the same reading device, e.g. during rounds, before the reading device's memory is downloaded into a data processing station. A system of bar codes can be used to keep track of the measuring devices from which information has been retrieved. Alternately, this can be accomplished when the physician records comments in the audio section, devised as a pocket memory.

## Claims

1. A method for transferring physiological data, obtained from a patient (3) with a stationary measuring device (2) placed near the patient, said data being stored in a memory (8) arranged in the measuring device, to a stationary data processing station (16), located at a distance from the measuring device, for processing and storage in a patient data base, **characterized in** that data stored in the measuring device (2) are retrieved with a portable reading device (10) and temporarily stored in a memory (14) arranged therein, whereupon the portable reading device is physically moved to or near the data processing station, and data stored in the reading device are downloaded into a memory (18) in the data processing station (16).

2. The system for transferring physiological data obtained from a patient to a stationary data processing station, located at a distance from the patient, comprising a stationary memory-equipped measuring device, located by the patient, for measuring and storing the said physiological data for subsequent transfer to the data processing station for processing and storage in a patient data base, **characterized in** that a portable, memory-equipped reading device for retrieving and temporarily storing data stored in the measuring device, said reading device being portable enough to be carried to or near the data processing station for downloading of data stored in the reading device to the data processing station.

3. The system according to claim 2, **characterized in** that the reading device contains an audio section with a microphone for recording information.

4. The system according to claim 3, **characterized in** that the memory of the reading device is digital, and an A/D converter is arranged to convert recorded verbal information into digital form for storage in the digital memory.

5. The system according to any of the claims 2-4, **characterized in** that the reading device is arranged to retrieve and temporarily store data from a plurality of measuring devices, the temporarily stored data from the various measuring device being transferable to the data processing station in a single, sequential downloading operation.

6. The system according to any of the claims 2-5, **characterized in** that the reading device's memory is a flash memory which retains stored information even when the reading device's source of energy is depleted or disabled.

7. The system according to any of the claims 2-6, **characterized in** that the reading device contains a transmitter/receiver unit, and the measuring device, transmitter/receiver unit and data processing station contain means for wireless input/output of data, preferably by telemetry or an IR link.

8. The system according to any of the claims 2-7, **characterized in** that the reading device is devised for loading in a rack in the data processing station, during the downloading of data to the data processing station, for simultaneous charging of the reading device's battery.

9. The system according to claim 8, **characterized in** that a coil is arranged to encircle the reading device, when it has been loaded in the rack in the data processing station, for charging of the reading device's battery by electromagnetic means by application of voltage to the coil.

10. The system according to claim 8, **characterized in** that the poles of the reading device's battery are connected to external contacts, intended, in turn, to make contact with charging contacts in the data processing station when the reading device is loaded in the rack.

11. The system according to any of the claims 2-10, **characterized in** that the reading device is combined with a penlight or devised as a pen-shaped implement.

12. The system according to any of claims 2-11, **characterized in** that the reading device is equipped with operating means for controlling input and output operations.
